# EUROPÄISCHE PATENTSCHRIFT

(11) **EP 1 483 051 B1**
(45) Veröffentlichungstag und Bekanntmachung des Hinweises auf die Patenterteilung: **14.12.2005**
(21) Anmeldenummer: 03743860.3
(22) Anmeldetag: 10.03.2003
(51) Int. Cl.: B01J 31/18, C07C 2/32, B01J 31/12, C07F 11/00, C07C 2/30, C07D 251/04

(54) **VERFAHREN ZUR OLIGOMERISIERUNG VON OLEFINEN UNTER VERWENDUNG EINES CYCLOALKYLALKYL-SUBSTITUIERTEN TRIAZACYCLOHEXANS**
METHOD FOR THE OLIGOMERIZATION OF OLEFINS USING A CYCLOALKYLALKYL-SUBSTITUTED TRIAZACYCLOHEXANE
PROCEDE D'OLIGOMERISATION D'OLEFINES UTILISANT UN TRIAZACYCLOHEXANE SUBSTITUE PAR CYCLOALKYLALKYLE

(30) Priorität: 14.03.2002 DE 10211386
(43) Veröffentlichungstag der Anmeldung: 08.12.2004
(73) Patentinhaber: Basell Polyolefine GmbH, 50389 Wesseling (DE)
(72) Erfinder: MIHAN, Shahram, 67061 Ludwigshafen (DE); MOLNAR, Ferenc, 67348 Speyer (DE); MAAS, Heiko, 68165 Mannheim (DE); PRINZ, Martina, 69221 Dossenheim (DE); KÖHN, Randolf, Bath BA2 2AZ (GB)
(86) Internationale Anmeldenummer: PCT/EP2003/002425
(87) Internationale Veröffentlichungsnummer: WO 2003/076367

(56) Entgegenhaltungen:
- WO-A-00/34211
- WO-A-00/58319
- KOEHN R D ET AL: "SELECTIVE TRIMERIZATION OF ALPHA-OLEFINS WITH TRIAZACYCLOHAXANE COMPLEXES OF CHROMIUM AS CATALYSTS" ANGEWANDTE CHEMIE. INTERNATIONAL EDITION, VERLAG CHEMIE. WEINHEIM, DE, Bd. 39, Nr. 23, 4. Dezember 2000 (2000-12-04), Seiten 4337-4339, XP000975912 ISSN: 0570-0833

## Beschreibung

Die vorliegende Erfindung betrifft ein Verfahren zur oligomerisierung von Olefinen sowie ein dafür geeignetes Katalysatorsystem.

Olefinoligomere mit bis zu 30 Kohlenstoffatomen haben große wirtschaftliche Bedeutung als Comonomere für Kunststoffe oder als Vorprodukte für Oxoalkohole, die ihrerseits Bestandteil von Tensiden und Weichmachern für Kunststoffe sind. Verfahren zur Oligomerisierung niederer Olefine, die z.B. Steamcrackern entstammen, kommt somit eine zentrale Bedeutung in der Herstellung von Produkten des täglichen Bedarfs zu.

Die WO 00/58319 beschreibt ein Verfahren zur Herstellung von Oligomeren von Olefinen unter Verwendung eines Oligomerisierungskatalysators, der aus einer Chromverbindung und einem 1,3,5-Triazacyclohexan sowie einem aktivierenden Zusatzstoff erhältlich ist.

Die JP-A-10/231317 offenbart ein Verfahren zur Herstellung von α-Olefinpolymeren unter Verwendung eines Vanadium- oder Chromkomplexes, einer mehrzähnigen Stickstoffverbindung und einer Alkylaluminiumverbindung.

Der Erfindung liegt die Aufgabe zugrunde, ein eingangs genanntes Verfahren anzugeben, das mit hoher Ausbeute zu Olefinoligomeren und vorzugsweise mit hoher Selekivität zu definierten Oligomeren, insbesondere Trimeren, führt.

Erfindungsgemäß wird diese Aufgabe gelöst durch ein Verfahren zur Oligomerisierung von olefinen, bei dem man ein Olefin mit einem Katalysatorsystem in Kontakt bringt, das erhältlich ist aus
a) wenigstens einer Chromquelle,
b) wenigstens einem Liganden der Formel I 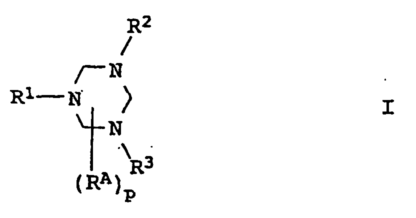 worin R¹ bis R³ unabhängig für Reste der Formel II 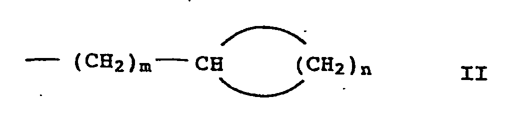 oder C₁- bis C₈-Alkyl stehen
   R^{A} unabhängig für C₁-C₈-Alkyl, C₅-C₇-Cycloalkyl ; C₆-C₁₅-Aryl, C₇-C₁₅-Arylalkyl oder einen Rest der Formel II steht, mit der Maßgabe, dass wenigstens einer der Reste R¹, R², R³ und R^{A} für einen Rest der Formel II steht,
   - p: für eine Zahl von 0 bis 6, vorzugsweise 0 bis 3,
   - m: für eine Zahl von 1 bis 6, vorzugsweise 1 bis 4,
   - n: für eine Zahl von 2 bis 6, vorzugseise 3 bis 5, steht, und
c) wenigstens einem Aktivator.

Die Erfindung betrifft auch das vorstehend definierte Katalysatorsystem.

In bevorzugten Ausführungsformen stehen R¹ bis R³ unabhängig für Reste der Formel II und p ist gleich O, oder die Reste R^{A} stehen für andere Reste als solche der Formel II, falls p von O verschieden ist.

In alternativen bevorzugten Ausführungsformen ist p von 0 verschieden und die Reste R^{A} stehen für solche der Formel II. Insbesondere steht p dann für 3 und die Reste RA sind symmetrisch am Triazacyclohexanring angeordnet, d. h. jedes Kohlenstoffatom im Triazocyclohexanring trägt einen Rest R^{A}. In diesem fall stehen R¹ bis R³ vorzugsweise für C₁- bis C₈-Alkyl, insbesondere Methyl oder Ethyl.

Vorzugsweise stehen R¹ bis R³ unabhängig für Cyclohexyl-C₁-C₄-alkyl, insbesondere für Cyclohexylmethyl, wobei p vorzugsweise für O steht.

Soweit R^{A} nicht für einen Rest der Formel IIsteht, steht dieser Rest unabhängig für C₁-C₁₈-Alkyl, vorzugsweise C₁-C₁₂-Alkyl wie Methyl, Ethyl, n-Propyl, i-Propyl, Butyl, Pentyl, Hexyl; C₅-C₇-Cycloalkyl, wie Cyclopentyl und Cyclohexyl, C₆-C₁₅-Aryl wie Phenyl, Methylphenyl oder Naphthyl; oder C₇-C₁₅-Arylalkyl, wie Benzyl.

Bevorzugte Liganden der Formel I sind 1,3,5-Tri-(cyclohexylmethyl)-1,3,5-triazacyclohexan, 1,3,5-Tri-(cyclohexylethyl)-1,3,4-triazacyclohexan und 1,3,5-Tri-(cyclohexylpropyl)-1,3,5-triazacyclohexan.

Die Liganden der allgemeinen Formel I, in denen p für 0 steht und die Reste R¹ bis R³ gleich sind, lassen sich in an sich bekannter Weise herstellen, insbesondere durch Umsetzung primärer Amine mit Formaldehyd oder Paraformaldehyd. Entsprechend sind Liganden der Formel I, die an den Kohlenstoffatomen des Triazacyclohexanringes Reste R^{A} tragen, aus primären Aminen und entsprechenden Aldehyden und/oder Ketonen erhältlich. Bezüglich geeigneter Herstellungsverfahren wird auf die WO 00/58319 und die darin zitierte Literatur verwiesen. Die beschriebenen Verfahren können analog zur Herstellung der Liganden der Formel I herangezogen werden.

Als Chromquelle eignen sich Chrom(II)- und/oder vorzugsweise Chrom(III)-Verbindungen. Geeignete Chrom(III)-Verbindungen sind insbesondere solche der Formel CrX₃, worin X für ein abstrahierbares Gegenion steht, insbesondere Halogen, wie Fluor, Brom, Jod und insbesondere Chlor; Tosylat, Triflat, Tetrafluoroborat, Hexafluorophosphat, Hexyfluoroantimonat, Tetraphenylborat; C₁-C₁₈-Carboxylat, wie Acetat, Butyrat, Neopentanoat, Laurat, Stearat oder 2-Ethylhexanoat. CrCl₃ hat sich besonders bewährt.

Als Chromquelle eignen sich auch Verbindungen der Formel CrX₃L₃, worin X die oben angegebene Bedeutung hat und L für einen neutralen Komplexliganden steht, z.B. Etherkomplexe, wie CrCl₃ (Tetrahydrofuran)₃, CrCl₃(Dioxan)₃, Esterkomplexe wie CrCl₃(n-Butylacetat), CrCl₃(Ethylacetat), Alkoholkomplexe wie CrCl₃ (i-Propanol)₃, CrCl₃(2-Ethylhexanol)₃, Aminkomplexe wie CrCl₃(Pyridin)₃, CrCl₃(i-Propylamin)₃, oder Nitrilkomplexe wie CrCl₃(Acetonitril)₃.

Aus der Chromquelle und dem Liganden der Formel I kann nach an sich bekannten Methoden (vgl. etwa W.A. Herrmann, A. Salzer: *"Synthetic Methods of Organometallic and inorganic Chemistry"* Vol. 1, Thieme Verlag, Stuttgart, 1996) ein Chromkomplex hergestellt werden, der isoliert und im erfindungsgemäßen Verfahren eingesetzt wird. Alternativ bringt man die Chromquelle und den Liganden der Formel I in situ im Reaktionsmedium in Kontakt. Der Ligand der Formel I wird in der Regel in wenigstens äquimolarer Menge, bezogen auf die Chromquelle (gerechnet als Chromatome) eingesetzt.

Als Aktivatoren kommen insbesondere Metallverbindungen mit wenigstens einer Metall-Kohlenstoff-Bindung in Betracht, die für die Zwecke der vorliegenden Anmeldung kollektiv als "Metallalkylverbindungen" bezeichnet werden. Repräsentative Metallalkylverbindungen sind Alkylaluminiumverbindungen, Alkylmagnesiumverbindungen, Alkylzinkverbindungen und/oder Alkyllithiumverbindungen. Davon sind Alkylaluminiumverbindungen bevorzugt. Sie können die Formeln AlR₃, AlR₂Hal, AlRHal₂, AlR₂OR', AlRHalOR' oder Al₂R₃Hal₃ aufweisen, worin R und R' unabhängig für Methyl, Ethyl oder eine geradkettige oder verzweigte C₃-C₈-Alkylgruppe stehen, und Hal für ein Halogenatom, wie Fluor, Brom, Jod oder insbesondere Chlor steht. Repräsentative Verbindungen sind Trimethylaluminium, Triethylaluminium, Tri-n-propylaluminium, Tri-iso-propylaluminium, Tributylaluminium, Diethylaluminiumchlorid, Diethylaluminiumbromid, Diethylaluminiumethoxid, Diethylaluminiumphenoxid und Ethylaluminiumethoxychlorid. Vorzugsweise werden Aluminiumalkylverbindungen vom Typ AlR₃ und AlRHal₂ eingesetzt, wobei Triethylaluminium oder ein Gemisch von Triethylaluminium und Ethylaluminiumdichlorid besonders bevorzugt sind.

Das molare Verhältnis von Chromquelle zur Aluminiumalkylverbindung beträgt üblicherweise 1:1 bis 1:100, vorzugsweise 1:5 bis 1:50.

Weitere geeignete Aktivatoren sind Alkylalumoxane, die beispielsweise aus der DE-A 3007725 bekannt sind. Es handelt sich um Produkte der partiellen Hydrolyse von Aluminiumalkylverbindungen. Alkylalumoxane können in Form linearer oder cyclischer Polymere vorkommen. Ihre Wirksamkeit als Aktivator erfüllen die Alkylalumoxane unabhängig von ihrer strukturellen Beschaffenheit. Das molare Verhältnis von Chromquelle zum Alkylalumoxan (gerechnet als Aluminiumatome) beträgt üblicherweise 1:1 bis 1:10000, vorzugsweise 1:1000.

In einer bevorzugten Ausführungsform des erfindungsgemäßen Verfahrens wird neben einer Aluminiumalkylverbindung als Aktivator ein gegebenenfalls substituierter fünfgliedriger aromatischer stickstoffhaltiger Heterocyclus mit verwendet. Geeignete fünfgliedrige aromatische stickstoffhaltige Heterocyclen sind solche mit 1,2,3 oder 4, vorzugsweise 1 oder 2 Stickstoffatomen im fünfgliedrigen aromatischen Ring. Die fünfgliedrigen aromatischen stickstoffhaltigen Heterocyclen können an den Ringkohlenstoffatomen durch unter den Reaktionsbedingungen inerte Gruppen wie Alkylgruppen, vorzugsweise Methyl- und/oder Ethyl, substituiert sein oder zwei benachbarte Kohlenstoffatome des fünfgliedrigen aromatischen stickstoffhaltigen Heterocyclus können einem ankondensierten aromatischen carbocyclischen System angehören, welches seinerseits inerte Gruppen tragen kann. Beispiele für derartige stickstoffhaltige Heterozyklen sind die Grundkörper und die substituierten Vertreter der Pyrrole, Pyrazole, Imidazole, Triazole und Tetrazole, wie Pyrrol, 2,5-Dimethylpyrrol, Indol, Carbazol, Pyrazol, Indazol, Imidazol, Benzimidazol. Vorzugsweise setzt man Pyrrole und insbesondere Alkyl-substituierte Pyrrole, vor allem 2,5-Dimethylpyrrol, ein.

Als weitere Co-Katalysatoren, die fakultativ mitverwendet werden können, eignen sich Alkylhalogenide, Alkylsiliziumhalogenide und Lewis-saure Metallhalogenide, vorzugsweise n-Butylchlorid, n-Butyliodid, Trimethylsilylchlorid, Trimethylsilylbromid, Zinntetrachlorid, Germaniumchlorid und vor allem n-Butylbromid.

In einer weiteren bevorzugten Ausführungsform des erfindungsgemäßen Verfahrens verwendet man neben einer Alkylaluminiumvesbindung als Aktivator mindestens eine Borverbindung. Geeignete Borverbindungen sind beispielsweise solche mit elektronenziehenden Resten, z.B. Trispentafluorphenylboran, N,N-Dimethylanilinium-tetrakis-(pentafluorphenyl)borat, Lithium-tetrakis-(pentafluorphenyl)borat, Tri-n-butylammonium-tetrakis-(pentafluorphenyl)borat, N,N-Dimethylanilinium-tetrakis-(3,5-bisperfluormethyl)-phenylborat, Tri-n-butylammonium-tetrakis-(3,5-bisperfluormethyl)-phenylborat sowie Tritylium-tetrakis-(pentafluorphenyl)borat. Derartige Borverbindungen sind aus der EP-A 468 537 sowie der EP-A 426 638 bekannt. Bevorzugt sind Tritylium-tetrakis-(pentafluorphenyl)borat, Trispentafluorphenylboran und insbesondere N,N-Dimethylanilinium-tetrakis-(pentafluorphenyl)-borat.

Das erfindungsgemäße Verfahren erfolgt in der Regel in flüssiger Phase in einem Lösungsmittel. Geeignete Lösungsmittel sind dabei aprotische Lösungsmittel, z.B. aliphatische gesättigte Kohlenwasserstoffe, wie Butan, Pentan, 3-Methylpentan, Hexan, Heptan, 2-Methylhexan, Octan, Cyclohexan, Methylcyclohexan, 2,2,4-Trimethylpentan, Decalin; halogenierte Kohlenwasserstoffe, wie Dichlorethan; aromatische Kohlenwasserstoffe, wie Benzol, Toluol, Xylol, Ethylbenzol, Mesitylen, Tetralin oder die unter Reaktionsbedingungen flüssigen oligomeren Reaktionsprodukte, wie 1-Hexen selbst eingesetzt werden. Diese Lösungsmittel können entweder einzeln oder als Gemisch verwendet werden.

Das erfindungsgemäße Verfahren eignet sich zur Oligomerisierung, insbesondere der selektiven Trimerisierung, von Ethen. Das erfindungsgemäße Verfahren eignet sich außerdem zur Oligomerisierung, insbesondere der selektiven Trimerisierung von α-Olefinen mit wenigstens drei Kohlenstoffatomen, wie 1-Propen, 1-Buten, 1-Hexen, 1-Decen. Als Olefin eignet sich insbesondere 1-Buten, gegebenenfalls im Gemisch mit seinen Isomeren, wie sie etwa im Raffinat II vorliegen.

Wegen der Hydrolyseneigung der als Aktivatoren eingesetzten Metallalkylverbindungen wird das erfindungsgemäße Verfahren in der Regel unter weitgehendem Feuchtigkeitsausschluss durchgeführt. Vorzugsweise arbeitet man unter Schutzgas. Als Schutzgase können alle unter Reaktionsbedingungen chemisch inerten Gase, wie Stickstoff oder Argon, verwendet werden. Daneben kann das umzusetzende Olefin selbst die Funktion des Schutzgases übernehmen, sofern es unter den Reaktionsbedingungen einen hinreichend hohen Dampfdruck hat.

Die Oligomerisierung wird vorzugsweise bei einer Temperatur im Bereich von 0 bis 120 und insbesondere 20 bis 110 °C durchgeführt. Sie erfolgt bei einem Druck von Umgebungsdruck bis 120 bar. Ein besonderer Vorteil des erfindungsgemäßen Verfahrens ist darin zu sehen, dass es bereits bei Umgebungsdruck, d.h. drucklos, zu guten Ausbeuten an Oligomerisierungsprodukten führt.

Nach beendeter Umsetzung wird das Katalysatorsystem in der Regel desaktiviert. Als Desaktivator eignet sich beispielsweise Wasser, das gegebenenfalls angesäuert ist, oder niedere Alkohole. Die Produkte der Oligomerisierung werden zweckmäßigerweise destillativ gereinigt. Nicht umgesetztes Ausgangsmaterial kann zurückgewonnen und in die Umsetzung zurückgeführt werden.

Die Erfindung wird durch die folgenden Beispiele näher veranschaulicht.

### Beispiel 1 bis 5: Drucklose Buten-Trimerisierung

In einem mit Kontaktthermometer, Rührer, Heizpilz und Gaseinleitungsrohr versehenen 1-Liter-Vierhalskolben wurden die in der nachstehenden Tabelle 1 angegebenen Chromkomplexe in 250 ml Toluol bei 40 °C vorgelegt. Methylalumoxan (MAO) wurde in Form einer 1,6 M-Lösung in Toluol verwendet. Das Atomverhältnis Cr:Al ist in der Tabelle angegeben. Durch die nach Zugabe von MAO erhaltene hellgrün/gelbe Lösung wurde 1-Buten geleitet.

Die Temperatur wurde 1 Stunde konstant bei 40 °C gehalten. Die Reaktion wurde durch Zugabe von 15 ml konzentrierter Salzsäure in 50 ml Methanol beendet und die Reaktionsmischung noch 15 Minuten nachgerührt. Danach wurden 250 ml Methanol zugegeben und weitere 15 Minuten gerührt. Nach Abfiltrieren wurde das Produkt dreimal mit Wasser gewaschen und über Natriumsulfat getrocknet. Ausbeute und Produktverteilung wurden gaschromatographisch aus der so erhaltenen Lösung bestimmt. Die Ergebnisse sind in der nachstehenden Tabelle 1 zusammengefasst.

**Tabelle 1**

| Beispiel | Komplex | Einwaage [µmol] | Atomverhältnis Cr:Al | Ausbeute Dodecen [g] | Aktivität [kg/(mol Cr*h)] |
|---|---|---|---|---|---|
| 1 * | (2-EtHex)₃TAC .CrCl₃ | 45,7 | 1:350 | 2,08 | 43 |
| 2 | (CyCH₂)₃ TAC.CrCl₃ | 31,5 | 1:350 | 4,63 | 147 |
| 3 | (CyCH₂CH₂)₃TAC .CrCl₃ | 44,3 | 1:350 | 3,74 | 84 |
| 4 | (CyCH₂CH₂CH₂)₃ TAC.CrCl₃ | 38,1 | 1:350 | 8,43 | 213 |
| 5* | (t-BuBz)₃TAC. CrCl₃ | 35,6 | 1:350 | 8,43 | 24 |

| | | | | | |
|---|---|---|---|---|---|
| * = Vergleichsbeispiel | | | | | |

### Beispiele 6 und 7: Drucklose Ethen-Trimerisierung

Die Versuchsdurchführung erfolgte wie bei den Beispielen 1 bis 5, wobei jedoch an Stelle von 1-Buten Ethen verwendet wurde. Die Ergebnisse sind in Tabelle 2 zusammengefasst.

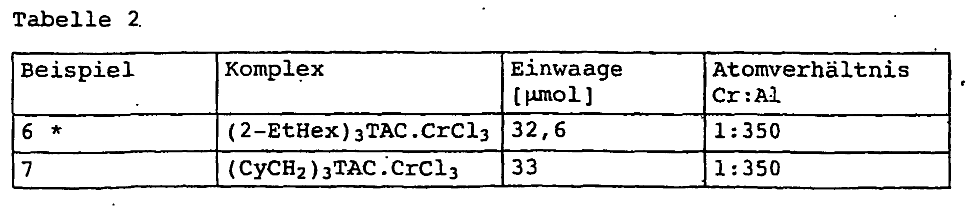

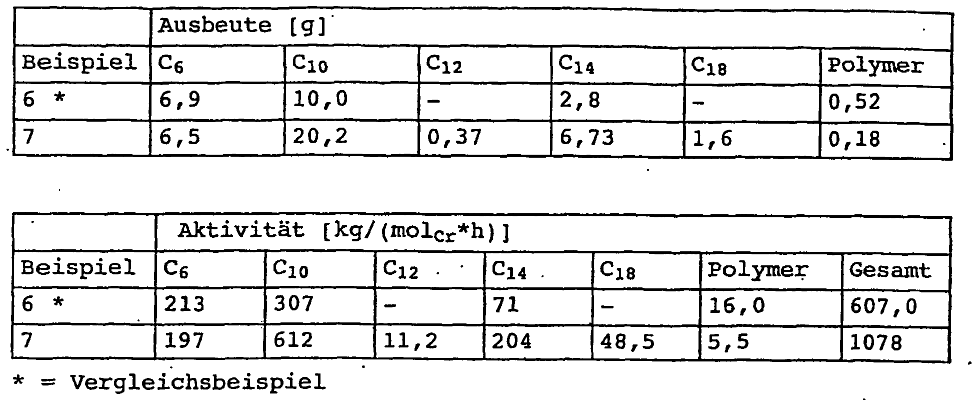

2-(Ethex)₃TAC =1,3,5-Tris(2-ethylhexyl)-1,3,5-triazacyclohexan (CyCH₂)₃TAC = 1,3,5-Tris(cyclohexylmethyl)-1,3,5-triazacyclohexan(CyCH₂CH₂)₃TAC = 1,3,5-Tris-(2-cyclohexylethyl)-1,3,5-triazacyclohexan

(C_{y}CH₂CH₂CH₂)₃TAC = 1,3,5-Tris(3-cyclohexylpropyl)-1,3,5-triazacyclohexan

(t-BuBz)₃TAC = 1,3,5-Tris-(p-t-butylbenzyl)-1,3,5-triazacyclohexan.

## Patentansprüche

1. Verfahren zur Oligomerisierung von Olefinen, bei dem man ein Olefin mit einem Katalysatorsystem in Kontakt bringt, das erhältlich ist aus
a) wenigstens einer Chromquelle,
b) wenigstens einem Liganden der Formel I 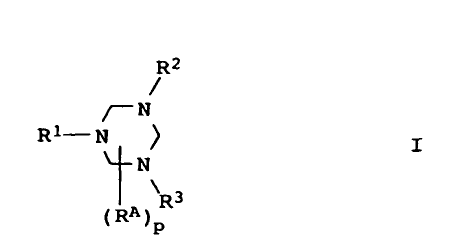 worin R¹ bis R³ unabhängig für Reste der Formel II 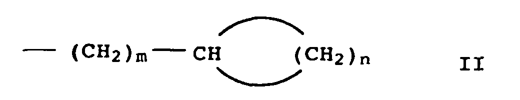 oder C₁- bis C₈-Alkyl stehen,
R^{A} unabhängig für C₁-C₁₈-Alkyl, C₅-C₇-Cycloalkyl, C₆-C₁₅-Aryl, C₇-C₁₅-Arylalkyl oder einen Rest der Formel II steht, mit der Maßgabe, dass wenigstens einer der Reste R¹, R², R³ und R^{A} für einen Rest der Formel II steht,
p für eine Zahl von 0 bis 6,
m für eine Zahl von 1 bis 6, und
n für eine Zahl von 2 bis 6 steht, und
c) wenigstens einem Aktivator.

2. Verfahren nach Anspruch 1, wobei R¹ bis R³ unabhängig für Reste der Formel II stehen und p gleich O ist, oder die Reste R^{A} für andere Reste als solche der Formel II stehen, falls p von O verschieden ist.

3. Verfahren nach Anspruch 1 oder 2, wobei R¹ bis R³ unabhängig für Cyclohexyl-C₁-C₄-alkyl stehen.

4. Verfahren nach Anspruch 3, wobei R¹ bis R³ für Cyclohexylmethyl stehen.

5. Verfahren nach Anspruch 1, wobei p für 3 steht und die Reste R^{A} symmetrisch am Triazacyclohexanring angeordnet sind und unabhängig für Reste der Formel II stehen.

6. Verfahren nach einem der Ansprüche 1 bis 5, wobei der Aktivator eine Alkylaluminiumverbindung umfasst.

7. Verfahren nach Anspruch 6, wobei der Aktivator ausgewählt ist unter AlR₃, AlR₂Hal, AlRHal₂, AlR₂OR', AlRHalOR' oder Al₂R₃Hal₃, worin R und R' unabhängig für Methyl, Ethyl oder eine gerakettige oder verzweigte C₃-C₈-Alkylgruppe und Hal für ein Halogenatom steht und Alkylalumoxanen.

8. Verfahren nach einem der Ansprüche 1 bis 7, wobei es sich bei dem Olefin um Ethen handelt.

9. Verfahren nach einem der Ansprüche 1 bis 7, wobei es sich bei dem Olefin um ein α-Olefin mit wenigstens 3 Kohlenstoffatomen handelt.

10. Katalysatorsystem, erhältlich aus
a) wenigstens einer Chromquelle,
b) wenigstens einem Liganden der Formel I, 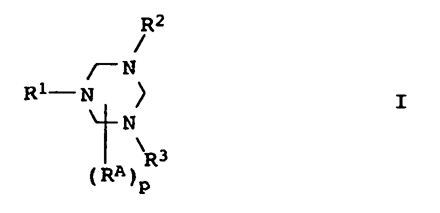 worin R¹ bis R³ unabhängig für Reste der Formel II 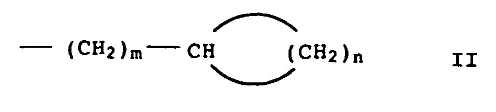 oder C₁- bis C₈-Alkyl stehen,
R^{A} unabhängig für C₁-C₁₈-Alkyl, C₅-C₇-Cycloalkyl, C₆-C₁₅-Aryl, C₇-C₁₅-Arylalkyl oder einen Rest der Formel II steht, mit der Maßgabe, dass wenigstens einer der Reste R¹, R², R³ und R^{A} für einen Rest der Formel II steht,
p für eine Zahl von 0 bis 6,
m für eine Zahl von 1 bis 6, und
n für eine Zahl von 2 bis 6 steht, und
c) wenigstens einem Aktivator.

## Claims

1. A process for the oligomerization of olefins in which an olefin is brought into contact with a catalyst system which is obtainable from
a) at least one chromium source,
b) at least one ligand of the formula I 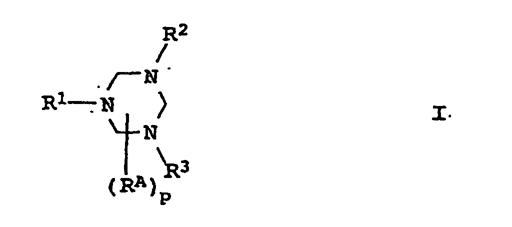 where R¹ to R³ are each, independently of one another, a radical of the formula II 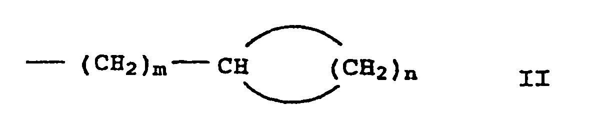 or C₁- to C₈-alkyl,
the radicals R^{A} are each, independently of one
another, C₁-C₁₈-alkyl, C₅-C₇-cycloalkyl, C₆-C₁₅-aryl,
C₇-C₁₅-arylalkyl or a radical of the formula II, with the proviso that at least one of the radicals R¹, R², R³ and R^{A} is a radical of the formula II,
p is from 0 to 6,
m is from 1 to 6,
n is from 2 to 6, and
c) at least one activator.

2. The process according to claim 1, wherein R¹ to R³ are, independently of one another, radicals of the formula II and p is 0, or the radicals R^{A} are radicals other than radicals of the formula II if p is different from 0.

3. The process according to claim 1 or 2, wherein R¹ to R³ are each, independently of one another, cyclohexyl-C₁-C₄-alkyl.

4. The process according to claim 3, wherein R¹ to R³ are each cyclohexylmethyl.

5. The process according to claim 1, wherein p is 3 and the radicals R^{A} are arranged symmetrically on the triazacyclohexane ring and are, independently of one another, radicals of the formula II.

6. The process according to any of claims 1 to 5, wherein the activator comprises an alkylaluminum compound.

7. The process according to claim 6, wherein the activator is selected from among AlR₃, AlR₂Hal, AlRHal₂, AlR₂OR', AlRHalOR' or Al₂R₃Hal₃, where R and R' are each, independently of one another, methyl, ethyl or a straight-chain or branched C₃-C₈-alkyl group and Hal is a halogen atom, and alkylaluminoxanes.

8. The process according to any of claims 1 to 7, wherein the olefin is ethene.

9. The process according to any of claims 1 to 7, wherein the olefin is an α-olefin having at least 3 carbon atoms.

10. A catalyst system obtainable from
a) at least one chromium source,
b) at least one ligand of the formula I 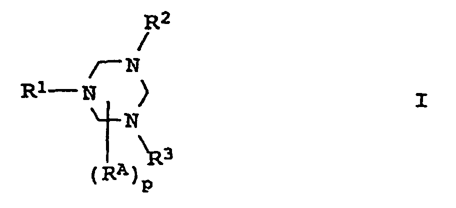 where R¹ to R³ are each, independently of one another, a radical of the formula II 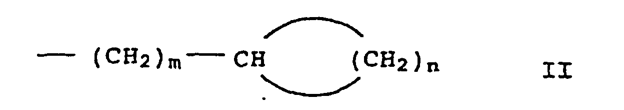 or C₁- to C₈-alkyl,
the radicals R^{A} are each, independently of one
another, C₁-C₁₈-alkyl, C₅-C₇-cycloalkyl, C₆-C₁₅-aryl, C₇-C₁₅-arylalkyl or a radical of the formula II, with the proviso that at least one of the radicals R¹, R², R³ and R^{A} is a radical of the formula II,
p is from 0 to 6,
m is from 1 to 6,
n is from 2 to 6, and
c) at least one activator.

## Revendications

1. Procédé pour l'oligomérisation d'oléfines, dans lequel on met une oléfine en contact avec un système catalyseur obtenu à partir de :
a) au moins une source de chrome,
b) au moins un ligand de formule I 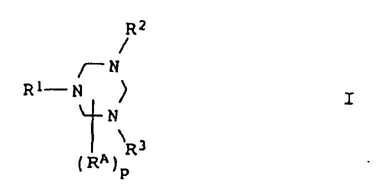 dans laquelle R¹ à R³ représentent, indépendamment, des groupes de formules II 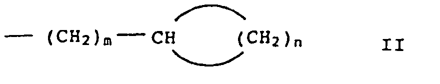 ou des groupes alkyle en C₁-C₈,
les symboles R^{A} représentent, indépendamment, des groupes alkyle en C₁-C₁₈, cycloalkyle en C₅-C₇, aryle en C₆-C₁₅, arylalkyle en C₇-C₁₅ ou un groupe de formule II, sous réserve qu'au moins un des symboles R¹, R², R³ et R^{A} représente un groupe de formule II,
p est un nombre allant de 0 à 6,
m est un nombre allant de 1 à 6, et
n est un nombre allant de 2 à 6, et
c) au moins un activateur.

2. Procédé selon la revendication 1, dans lequel R¹ à R³ représentent, indépendamment, des groupes de formule II et p est égal à 0, ou bien les symboles R^{A} représentent des groupes autres que ceux de formule II lorsque p est différent de 0.

3. Procédé selon la revendication 1 ou 2, dans lequel R¹ à R³ représentent, indépendamment, des groupes cyclohexyl-alkyle en C₁-C₄.

4. Procédé selon la revendication 3, dans lequel R¹ à R³ représentent des groupes cyclohexylméthyle.

5. Procédé selon la revendication 1, dans lequel p est égal à 3 et les groupes R^{A} sont en disposition symétrique sur le cycle triazacyclohexane et consistent, indépendamment, en groupes de formule II.

6. Procédé selon une des revendications 1 à 5, dans lequel l'activateur est un dérivé d'alkylaluminium.

7. Procédé selon la revendication 6, dans lequel l'activateur est choisi parmi AIR₃, AIR₂Hal, AlRHal₂, AlR₂OR', AlRHalOR' ou Al₂R₃Hal₃, R et R' représentant, indépendamment des groupes méthyle, éthyle ou alkyle à chaîne droite ou ramifiée en C₃-C₈ et Hal un atome d'halogène, et les alkylalumoxanes.

8. Procédé selon une des revendications 1 à 7, dans lequel l'oléfine est l'éthène.

9. Procédé selon une des revendications 1 à 7, dans lequel l'oléfine est une α-oléfine à au moins 3 atomes de carbone.

10. Système catalyseur obtenu à partir de :
a) au moins une source de chrome,
b) au moins un ligand de formule I, 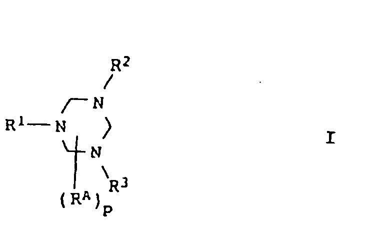 dans laquelle R¹ à R³ représentent, indépendamment, des groupes de formules II 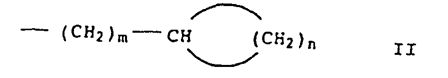 ou des groupes alkyle en C₁-C₈,
les symboles R^{A} représentent, indépendamment, des groupes alkyle en C₁-C₁₈, cycloalkyle en C₅-C₇, aryle en C₆-C₁₅, arylalkyle en C₇-C₁₅ ou des groupes de formule II, sous réserve qu'au moins un des symboles R¹, R², R³ et R^{A} représente un groupe de formule II,
p est un nombre allant de 0 à 6,
m est un nombre allant de 1 à 6, et
n est un nombre allant de 2 à 6, et
c) au moins un activateur.
